# EUROPEAN PATENT SPECIFICATION

(11) **EP 0 409 315 B1**
(45) Date of publication and mention of the grant of the patent: **14.05.1997**
(21) Application number: 90201863.9
(22) Date of filing: 10.07.1990
(51) Int. Cl.: B32B 31/08, B31F 1/22

(54) **Improved method for manufacturing a laminate having at least ione pleated lamina**
Verfahren zum Herstellen eines Laminats mit mindestens einer gewellten Schicht
Procédé pour la fabrication d'un produit stratifié avec au moins une couche ondulée

(30) Priority: 19.07.1989 US 382516
(43) Date of publication of application: 23.01.1991
(73) Proprietor: THE PROCTER & GAMBLE COMPANY, Cincinnati, Ohio 45202 (US)
(72) Inventor: Goulait, David Joseph Kenneth, Cincinnnati, Ohio 45241 (US); Carstens, Jerry Edward, Mason, Ohio 45040 (US)
(74) Representative: Bottema, Johan Jan

(56) References cited:
- FR-A- 2 088 069
- US-A- 2 547 880
- US-A- 3 425 888
- US-A- 4 377 431

## Description

### TECHNICAL FIELD

The present invention relates to a method of manufacturing a laminate having at least two laminae, at least one lamina of which is pleated in the machine direction. More particularly the invention relates to a method of manufacturing such a laminate by using an apparatus having at least one circumferentially grooved roll.

### BACKGROUND OF THE INVENTION

Laminates having at least one pleated lamina are known in the prior art. For example, U.S. Patent 4,377,431, issued March 22, 1983, to Chodosh teaches a fabric having three substantially coextensive laminae, one lamina of which is pleated. The prior art also teaches the use of rolls 22 and 24 having circumferential grooves and lands to impart lateral stretch to a lamina, as, for example, shown by U.S. Patent 4,517,714, issued May 21, 1985, to Sneed et al. The prior art further discloses a method for forming laminated cellular units. This is contained in US Patent 2,547,880 issued August 9, 1949, to Meyer et al.

However, the prior art does not show a method to join at least two laminae in face to face relation, at least one lamina being pleated, using a process which does not provide significant constraints upon the speed of such joining method. In fact, FR 2,088,069 issued May 12, 1970 to Guichard does not teach a high speed process. Therefore, it is an object of this invention to provide a method of joining at least two laminae, at least one of which is pleated, in face to face relation, at relatively high speeds (e.g. greater than 120 meters per minute) and to do so using conventional and known equipment. It is further an object of this invention to provide a method which can produce a laminate having one pleated lamina or two pleated and oppositely facing laminae.

### SUMMARY OF THE INVENTION

The present invention is a method for joining two laminae, at least one of which is pleated, in face to face relation, at least one lamina having pleats each lamina comprising a flexible woven or non-woven material, a film material or a paper sheet material. The method comprises the steps of providing two laminae, at least one lamina being pleated, and two rolls having mutually parallel axes and therebetween defining a nip. At least one of the rolls has a plurality of circumferential grooves and circumferential lands intermediate the grooves. The laminae are passed through the nip in face to face relation with the pleated lamina facing the grooved roll. The pleats of the pleated lamina are transversely registered with the grooves of the grooved roll. The rolls are then pressed together about the plane generally perpendicular to and connecting the axes of the rolls to effect joining of the laminae.

In one execution, a smooth lamina and a pleated lamina may be provided and joined in face to face relation by passing through the laminae the nip defined by a smooth roll and a grooved roll. In a second execution, two pleated laminae are joined in face to face relation by passing the laminae through the nip defined by two circumferentially grooved rolls.

### BRIEF DESCRIPTION OF THE DRAWINGS

While the Specification concludes with claims particularly pointing and distinctly claiming the present invention, it is believed the same will be better understood by the following description taken in conjunction with the accompanying drawings in which like parts are designated by the same reference numeral, related or analogous parts are designated with a prime symbol and:
Figure 1 is a vertical sectional view of a laminate produced according to the present invention and having one pleated lamina and one unpleated lamina;
Figure 2 is a vertical sectional view of an apparatus used to produce the laminate of Figure 1, as viewed in the machine direction;
Figure 3 is an enlarged view of a portion of the apparatus of Figure 2, showing the grooves and lands;
Figure 4 is a vertical sectional view of a laminate produce according to the present invention and having two pleated laminae;
Figure 5 is a vertical sectional view of an apparatus used to produce the laminate of Figure 4, as viewed in the machine direction;
Figure 6 is a vertical sectional view of a laminate produce according to the present invention having one pleated lamina of nonuniform pitch; and
Figure 7 is a vertical sectional view of an apparatus used to produce the laminate of Figure 6, as viewed in the machine direction.

### DETAILED DESCRIPTION OF THE INVENTION

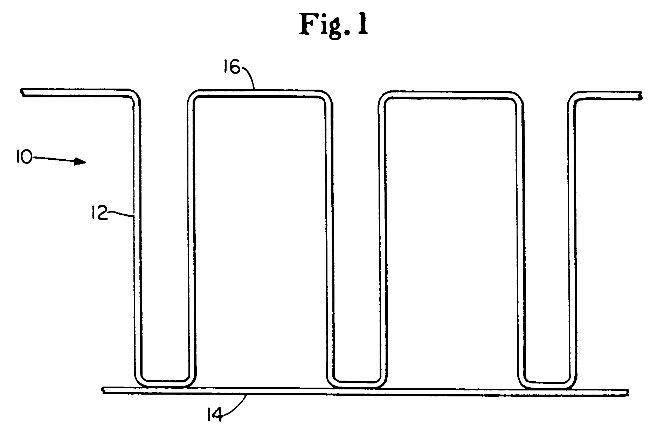

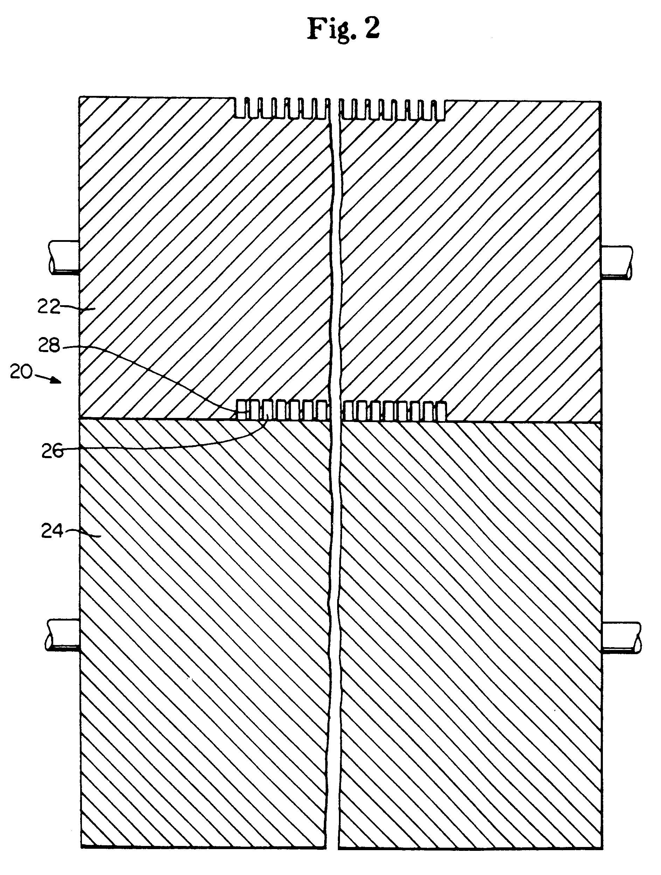

The present invention is directed to joining two laminae in face to face relation to produce a unitary laminate 10, as illustrated in Figure 1. The first lamina 12 of the laminate 10 is pleated with pleats 16 oriented in the machine direction and the second lamina 14 is unpleated. The laminate 10 may be produced with the apparatus 20 illustrated by Figure 2. The apparatus 20 comprises two rolls 22 and 24 and the nip defined therebetween.

Preferably, the rolls 22 and 24 have generally straight axes, although rolls 22 and 24 having curved axes (not shown) may be used with the present invention as well. The rolls 22 and 24 are disposed and rigidly held by a frame (not shown) as is commonly known in the art, so that the axes of the rolls 22 and 24 are mutually parallel. The frame may support both ends of the rolls 22 and 24, or the rolls 22 and 24 may be cantilevered from one end.

The first roll 22 is circumferentially grooved and the second roll 24 has a generally smooth, uninterrupted circumference. As used herein, the term "groove" refers to a channel in the roll 22, which channel extends substantially around the circumference of the roll 22. The bottom of the groove 26 is that face of the groove 26 which is of the least diameter. The sides of the groove 26 are those faces radially oriented and which extend from the bottom of the groove 26 to the outer circumference of the roll 22. The groove 26 may be of any suitable cross section, although a rectangular cross section, as shown, is generally, preferred.

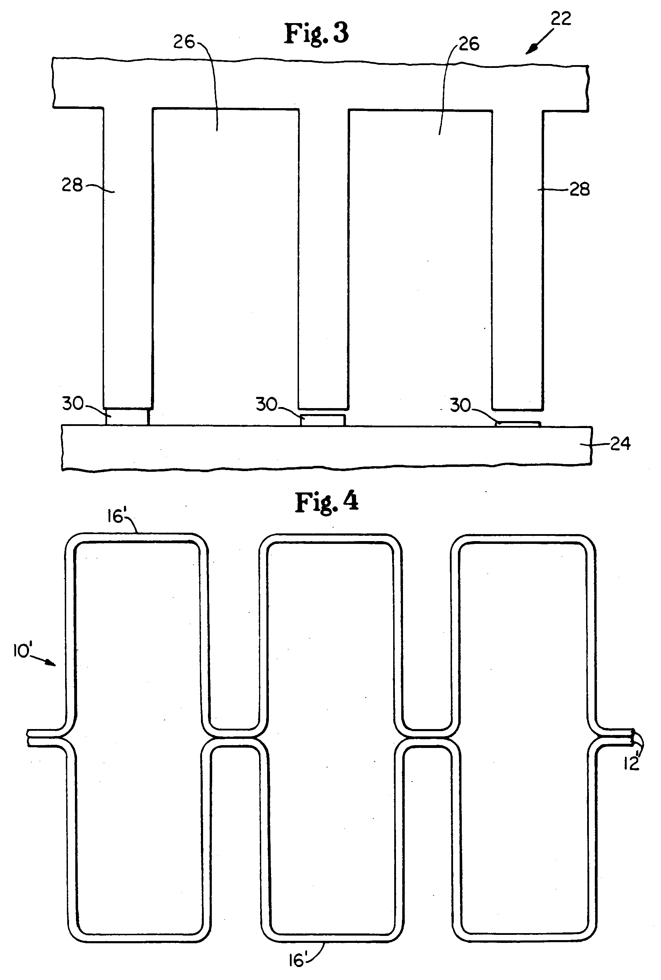

As illustrated in Figure 3, intermediate the grooves are lands 28. As used herein, the phrase "land" refers to any portion of the roll 22 intermediate two grooves 26 and which has a diameter greater than that than of the bottom of either adjacent groove 26. Each land 28 may be thought of as an annular cantilevered beam having a fixed end at the bottom of the grooves 26 and a free end at the outer circumference of the roll 22. It is not necessary that the land 28 be of constant diameter throughout the entire circumference. It is only necessary that the space between the grooves 26 be interrupted in diameter and that the grooves 26 are not contiguous. As used herein the "depth" of the groove 26 is the difference between the radius at the bottom of the groove 26 and the greatest radius of the outermost portion of the adjacent land 28. As used herein the term "pitch" refers to the distance, taken parallel to the axis of the roll 22, between the circumferential centerlines of adjacent grooves 26, or the distance between centerlines of adjacent pleats 16 of the pleated lamina 12.

Disposed on the distal end of the land 28 of the grooved roll 22 or on the periphery of the smooth roll 24 and registered with the lands 28 of the grooved roll 22 may be one or more generally radially oriented upstanding protrusions 30. The upstanding protrusions 30 may be generally cylindrically or rectangularly shaped, and provide discrete bonding points for the laminae 12 and 14 passing through the nip. The protrusions 30 have a height of about 0.5 millimeters (0.020 inches) and a diameter of about 0.5 millimeters to about 1.4 millimeters (0.20 inches). The rolls 22 and 24 are pressed together to effect joining the laminae 12 and 14. The protrusions 30 provide sites of increased compressive pressure, so that a more stable bond is obtained. Preferably, the protrusions 30 are not circumferentially registered, so that the bonding sights are transversely staggered on the resulting laminate 10.

The diameters of the rolls 22 and 24 are controlled by the compressive force used to urge the rolls 22 and 24 together and the length of the rolls 22 and 24. As either parameter increases, the minimum diameter of the grooved roll 22 (measured at the bottom of the grooves 26) must increase accordingly. The diameter of the roll 22 at the outer periphery of the lands 28 is then selected in accordance with the desired pleat height. The diameter of the second roll 24 is preferably different from, but no less than, the minimum diameter of the grooved roll 22 - so that adequate strength is maintained, but the same points on the circumferences of the rolls 22 and 24 do not contact on each revolution. Generally as the difference between the diameter of the roll 22 at the bottom of the grooves 26 and the diameter at the circumference of the lands 28 increases, the lands 28 should be made wider to provide adequate strength and to preclude axial movement of the distal ends of the lands 28.

The width and pitch of the grooves 26 of the grooved roll 22 should correspond generally to the width and pitch of the pleats 16 of the pleated lamina 12. For pleats having a pitch less than about 4.3 millimeters (0.17 inches), the grooves 26 may be about 10 percent wider than the desired pleats 16 to allow for variations in tracking and volume of the pleated lamina 12. Further, the grooves 26 should have a depth sufficient to accommodate the size of the pleats 16, otherwise wrinkling of the pleated lamina 12 may occur.

Pleat sizes ranging in height from about 0.8 millimeters to about 19.05 millimeters (0.03 inches to 0.75 inches) and grooves 26 having a pitch ranging from about 2.5 millimeters to 13 millimeters (0.10 inches to 0.50 inches) work well with the method disclosed herein. Thus the grooves 26 may advantageously be made of any size within this range, or of other sizes and geometries as desired.

The second roll 24 is relatively smooth, being ungrooved, corresponding to the second lamina 14 of the laminate 10. Generally as the force compressing the first and second rolls 22 and 24 increases, or the length of the rolls 22 and 24 increases, a larger diameter second roll 24 is desired. Alternatively, the second 24 roll may be slightly bowed towards the first 22 roll so that constant surface interface is maintained between the rolls 22 and 24.

Each roll 22 and 24 is free to rotate about its respective axis. The rolls 22 and 24 rotate in the opposite sense - so the circumferences of the rolls 22 and 24 are traveling in substantially the same direction at the nip. Preferably, both rolls 22 and 24 are externally driven, particularly as higher processing velocities are utilized. Alternatively, the first roll 22 may be externally driven, and the second roll 24 rotated by the tangential frictional component of the laminae 12 and 14 passing through the nip. The laminae 12 and 14 may pass through nip at a velocity of about 120 to about 310 meters per minute (400 to 1,000 feet per minute). For speeds of about 120 meters per minute, or greater, a motor of about 7500 watts for each roll 22 and 24 is suitable. It is not, however, necessary that the surface velocity of the rolls 22 and 24 be matched.

The laminae 12 and 14 are held in face to face relation and pass through the nip in the machine direction. As used herein, the term "machine direction" refers to that direction generally perpendicular to both axes of the rolls 22 and 24 and the plane which connects the axes of the rolls 22 and 24. Furthermore, as used herein, the term "cross machine direction" refers to the direction generally parallel to the axes of the rolls 22 and 24 and perpendicular to the machine direction. The "nip plane" is that plane generally perpendicular to the plane which connects the axes of the rolls 22 and 24 and which intercepts the nip of the rolls 22 and 24.

The laminae 12 and 14 may be composed of any material suitable for the end-use application. Generally, as the thickness of the pleated lamina 12 increases, the pitch of the grooves 26 should increase to accommodate the greater amount of material present. The process described herein is successfully used with elastomeric, paper and polyolefinic laminae 12 and 14, such as polypropylene and polyethylene. The pleated lamina 12 may have a thickness ranging from about 0.02 millimeters to about 0.5 millimeters (0.0008 inches to 0.02 inches). The pleated lamina 12 may be either a film or a nonwoven material. The unpleated lamina 14 may be elastomeric. The unpleated lamina 14 may be a nonwoven material, but is preferably a film.

The first lamina 12 is pleated, using any means well known in the art for providing "pleats", i.e. portions displaced from the plane of the lamina 12, which pleats are oriented substantially parallel to the machine direction. Suitable pleating methods are disclosed in U.S. Patent 4,252,591, issued February 24, 1981 to Rosenburg, and Canadian Patent 758,794, issued May 16, 1967 to Ives et al. The second lamina 14 is used in a relatively smooth, unpleated condition.

The laminae 12 and 14 are preferably supplied from individual supply rolls (not shown). Each supply roll is mounted with its respective axis generally parallel that of the rolls 22 and 24 and upstream of the nip. The laminae 12 and 14 are brought together in face to face relation either by using tracking rolls (not shown) or directly through the nip of the rolls 22 and 24. The laminae 12 and 14 may be pulled through the nip by takeup rolls 22 and 24 (not shown). The laminae 12 and 14 may pass through the nip at the same surface velocity or, if desired, at a different surface velocity,

The pleated lamina 12 is transversely registered with the grooves 26 so that preferably the centerline of each pleat 16 is aligned with the centerline of each groove 26. This may be accomplished by transversely adjusting the position of the supply roll, or the position of the last roll which the pleated lamina 12 crosses, relative to the position of the grooved roll 22. Such adjustment may be accomplished by axial movement of either the supply roll or the grooved roll 22. It is important that the pleated lamina 12 travel through the nip in a direction parallel to the machine direction, otherwise wrinkling and bunching of the lamina 12 within the grooves 26 and at the nip may result. Therefore, the axis of the supply over for the pleated lamina 12 should be generally parallel to the nip.

It is important that the pleated lamina 12 not become wrinkled or transversely misregistered with the grooves 26 of the grooved roll 22. If this occurs, the pleats 16 may become laterally tensioned and a laminate 10 of uniform pitch and the desired aesthetics may not result. Typically, if the pleats 16 should become laterally tensioned (e.g. due to a geometry mismatch with the grooves 26 of the first roll 22 or not being registered with the grooves 26) a relatively large pleat 16 will result at the point the pleated lamina 12 was tensioned and a relatively small pleat 16 will result elsewhere, due to the relative loss and transfer of material necessary to form the larger pleat 16.

The position of the unpleated lamina 14 relative to the nip is not critical. It is only necessary that the transverse ends of the laminae 12 and 14 be coterminous, otherwise the resulting laminate 10 will have a single lamina at each edge. Although it is not critical, the unpleated lamina 14 should pass through the nip generally parallel to the machine direction, otherwise the unpleated lamina 14 may not be properly joined to the pleated lamina 12.

The confluent laminae 12 and 14 are joined at the nip or after passing through the nip. The laminae 12 and 14 may be joined by adhesive or autogenous bonding. If adhesive bonding is selected, the adhesive is applied to the face of either lamina 12 or 14 which is oriented towards the other lamina 12 or 14. Alternatively, an adhesive lamina (not shown) may be interposed between the pleated 12 and unpleated lamina 14 so that a laminate 10 having three laminae is formed; with a central lamina of adhesive and two outboard laminae 12 and 14, one pleated, one unpleated, as described above. One suitable method for producing such a three laminae laminate 10 is disclosed in U.S. Patent 4,377,431 issued March 22, 1983, to Chodosh. Pressure sensitive adhesive marketed by The Century Adhesives Company of Columbus, Ohio as a diaper chassis adhesive has been found to work well for adhesive joining of the laminae 12 and 14.

Alternatively, a three (or more) laminae laminate 10 having a nonadhesive central lamina may be formed. If a nonadhesive central lamina is selected, the three (or more) laminae may be joined by autogenous bonding.

If autogenous bonding is selected, it may be accomplished by heating the rolls 22 and 24 and pressing the rolls 22 and 24 together about the plane generally perpendicular to and connecting the axes of the rolls 22 and 24. Additionally, the laminae 12 and 14 may be drawn through the nip at a differential velocity of about 2% to about 40% of the velocity of the lamina 12 or 14 having the lower velocity at the plane of the nip. Autogenous bonding, as described in EP 295957A No., granted December 21, 1988 in the name of Ball et al., has been found suitable.

The rolls 22 and 24 may be pressed together so that a pressure of about 42,200,000 to about 56,200,000 kilograms per square meter (60,000 to 80,000 pounds per square inch) is obtained and the laminae 12 and 14 are brought into contacting relationship at the positions of the lands 28. The pressure is measured by dividing the compressive force applied to the rolls 22 and 24 by the area of the protrusions 30 at the bonding footprint. The bonding footprint is obtained by inserting an unpleated stationary lamina 14 between the rolls 22 and 24 and compressing the rolls 22 and 24 with a known force until an impression on the lamina 14 is obtained from each protrusion in the vicinity of the nip. The area of each protrusion and the number of impressions are counted and summed to yield the effective bonding area. This area is divided into the applied force to yield the compressive pressure on the roll 22 or 24 having the protrusions 30.

A nonlimiting example of the process disclosed herein found suitable for producing a laminate 10 having two laminae 12 and 14, one pleated, one unpleated is as follows. A first lamina 12 having pleats of about 9.5 millimeters (0.38 inches) height, about 4.7 millimeters (0.19 inches) width, and a pitch of about 6.4 millimeters (0.25 inches) is provided. The second lamina 14 is unpleated and generally smooth. Both laminae 12 and 14 are made of a thermoplastic material, particularly polypropylene. The first lamina 12 is a polypropylene nonwoven material of about 2.42 dtex (about 2.2 denier) and a basis weight of about 24 grams per square meter (20 grams per square yard). The second lamina 14 is a polypropylene film having a thickness of about 0.2 millimeters (0.008 inches).

Two rolls 22 and 24 are provided. The first roll 22 is grooved, with grooves 26 of about 9.5 millimeters (0.38 inches) in depth, about 4.7 millimeters (0.19 inches) in width and of about 6.4 millimeters (0.25 inches) in pitch. The maximum diameter of the roll 22 is about 15.2 centimeters (6 inches) and the diameter at the bottom 26 of the grooves is about 14.0 centimeters (5.5 inches). The second roll 24 is not grooved and has a diameter of about 29 centimeters (11.4 inches). Both rolls 22 and 24 have straight and mutually parallel axes, and are about 33 centimeters (13 inches) long.

The second roll 24 has 36 equally spaced protrusions 30 corresponding to the axial position of each land. Each protrusion 30 is about 3.8 millimeters (0.015 inches) in radial dimension and has a parallelogram shaped surface of about 1.9 square millimeters (0.003 square inches). The rolls 22 and 24 are pressed together with a force of about 2500 kilograms (5600 pounds), so that a pressure of about 49,200,000 kilograms per square meter (70,000 pounds per square inch) is obtained on the raised protrusions 30 of the circumferentially grooved roll 22.

The laminae 12 and 14 are drawn through the nip defined by the rolls 22 and 24 at a uniform and constant velocity of about 180 meters per minute (600 feet per minute). Both rolls 22 and 24 are heated to a surface temperature of about 82°C to provide for autogenous bonding of the laminae 12 and 14. The resulting laminate 10 has pleats 16 of generally uniform pitch, height, and width.

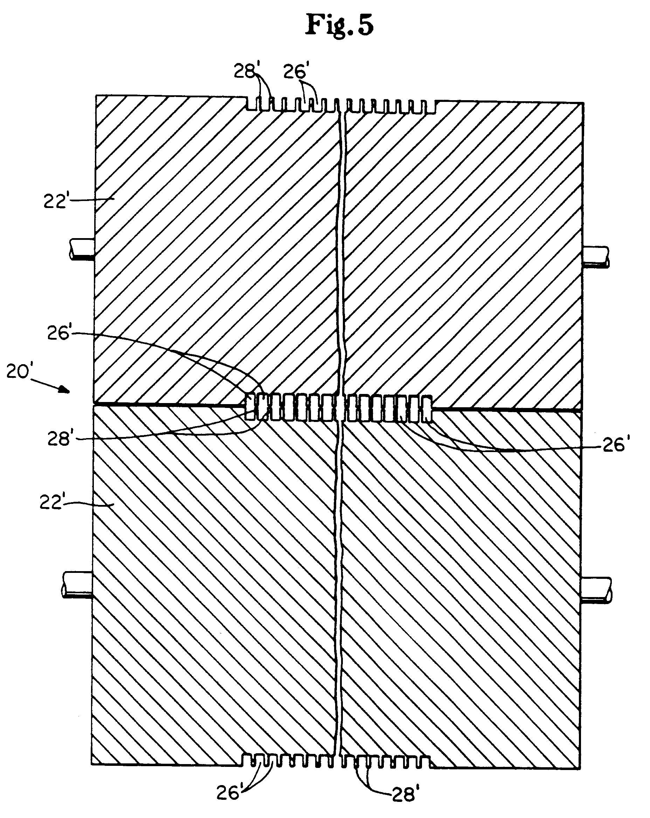

Variations in the disclosed method of producing a pleated laminate 10 are feasible. For example, as shown in Figure 4, a laminate 10 having two pleated laminae 12′, each with oppositely facing machine direction oriented pleats 16′ can be produced by the method described herein. Referring to Figure 5, to produce such a laminate 10′, it is only necessary two grooved rolls 22′ and the nip defined therebetween, be provided, and that at least two, and preferably more, of the lands 28′ of the grooved rolls 22′ be transversely registered.

Two laminae 12′ having longitudinal pleats made of either the same or different materials, and formed as described above, are provided and confluently passed through the nip in face to face relation with the pleated surfaces of the laminae 12′ facing outwardly and oppositely from each other. The pleats 16′ of the laminae 12′ are matched in geometry to the circumferential grooves 26′ of the rolls 22′ and transversely registered as described above. The laminae 12′ are then joined, either adhesively or autogenously as described above.

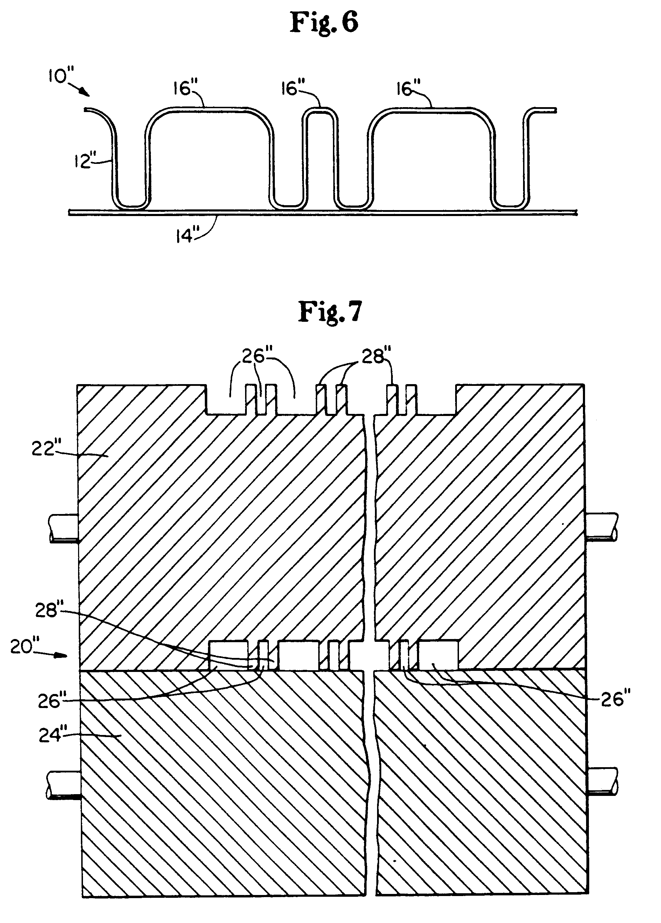

In another variation of the method of making the first above-described laminate 10, a laminate 10˝ having a pleated lamina 12˝ of nonuniform pitch, and a unpleated lamina 14˝ results, as illustrated in Figure 6. Referring to Figure 7, to produce such a laminate 10˝, a circumferentially grooved roll 22˝ having a nonuniform pitch, i.e. the spacing between adjacent grooves 26˝ varies along the axial length of the roll 22˝, is provided. A roll 24˝ which is not grooved is also provided. A first lamina 14˝ is provided, having pleats 16˝ of nonuniform pitch, corresponding to the width and pitch of the grooves 26˝ of the first roll 22˝. The first lamina 12˝ and a second unpleated lamina 14˝ are confluently passed through the nip in face to face relation as described above. This process yields laminate 10˝ having one lamina 12˝ with spaced pleats 16˝ of a nonuniform and variable pitch and one unpleated lamina 14˝. Thus, the grooves 26˝ of the grooved roll 22˝ may either be spaced on a uniform pitch or on a nonuniform pitch.

It will be apparent to one skilled in the art, that the two variations described above may be combined to produce a laminate 10′ having two laminae 12′ with outwardly facing pleats 16′ of a different but uniform pitch or having one or two nonuniform pitches. This execution may be combined with the three laminae laminate 10, discussed above, so that a laminate 10′ having two pleated outboard laminae 12′ and a central unpleated lamina results.

Another feasible variation is to utilize elastically extensible laminae 12 and particularly 14, extensible perpendicular to the pleats 16, so that the resulting laminate 10 has elastic properties and may be stretched. A preferred elastically extensible embodiment has an unpleated elastomeric lamina 14 joined to a pleated lamina 12. The unpleated lamina 14 may be stretched either before or after being joined to the pleated lamina 12. Another preferred elastically extensible embodiment has two outboard pleated laminae 12' and a central elastomeric unpleated lamina.

If the unpleated lamina 14 is stretched in the machine direction prior to bonding, or if the pleated and unpleated laminae 12 and 14 or both pleated laminae 12' are extensible in the direction generally parallel to the pleats 16, a laminate 10 or 10' having bielastic properties may be produced and stretched parallel or perpendicular to the pleats 16.

## Claims

1. A method of joining two laminae in face to face relation, at least one lamina having pleats each lamina comprising a flexible woven or non-woven material, a film material or a paper sheet material, said method comprising the steps of:
providing a first lamina and a second lamina, at least said first lamina having pleats;
providing a first roll and a second roll having mutually parallel axes and defining a nip therebetween, at least said first roll having a plurality of circumferential grooves and lands intermediate said grooves;
passing said laminae through said nip in face to face relation, whereby said first lamina faces said first roll;
transversely registering the pleats of said pleated lamina with said grooves of said grooved roll;
pressing said rolls together so that said lamina contact each other at a position corresponding to said lands for bonding said laminae together at said positions of contact.

2. A method according to claim 1, wherein the second lamina is unpleated and wherein the second roll does not have circumferential grooves and lands;
the method comprising the step of:
passing said laminae through said nip in face to face relation, whereby said first lamina faces said first roll and said second lamina faces said second roll.

3. A method according to claim 1, each lamina having pleats, said pleats of said laminae being substantially mutually parallel and outwardly facing, wherein each said roll has a plurality of circumferential grooves and lands intermediate said grooves, at least one land of said first roll being transversely registered with at least one land of said second roll, the method comprising the steps of:
passing said laminae through said nip in face to face relation, whereby said pleats are oppositely disposed and said pleats of said first lamina face said grooves of said first roll and said pleats of said second lamina face said grooves of said second roll; and
transversely registering the pleats of said first lamina with said grooves of said first roll and the pleats of said second lamina with said grooves of said second roll.

4. The method of Claims 1, 2 or 3 wherein at least one of said laminae are passed through said nip at a velocity of about 120 meters per minute to about 310 meters per minute.

5. The method of Claim 4 wherein said laminae pass through said nip at a velocity differential of at least about 2 % of the velocity of the lamina having the lesser velocity.

6. The method of Claims 1, 2 or 3 wherein said rolls 22 and 24 are pressed together so that a pressure of about 42,200,000 kilograms per square meter to about 56,200,000 kilograms per square meter is obtained on at least one roll.

7. The method of Claims 1, 2 or 3 further comprising the step of heating at least one of said rolls to a temperature sufficient to effect joining of said laminae.

8. The method of Claims 1, 2 or 3 wherein each of said grooved rolls have a pitch selected from the group consisting of uniform spacing and non uniform spacing.

9. The method of Claims 1, 2 or 3 further comprising the step of interposing a third lamina between said first lamina and said second lamina, so that a laminate having three laminae joined in face to face relation is formed.

10. The method of Claims 1, 2 or 3 further comprising the step of stretching at least one of said laminae.

## Patentansprüche

1. Ein Verfahren zum Verbinden zweier dünner Blätter in Gegenüberstellungsbeziehung, wobei mindestens ein dünnes Blatt Falten aufweist, jedes dünne Blatt ein flexibles gewebtes oder Faservlies-Material, ein Folienmaterial oder ein Papierblatt-Material umfaßt, wobei das genannte Verfahren folgende Schritte umfaßt:
Beistellen eines ersten dünnen Blattes und eines zweiten dünnen Blattes, wobei mindestens das genannte erste dünne Blatt Falten aufweist;
Beistellen einer ersten Walze und einer zweiten Walze, welche zueinander parallele Achsen aufweisen und dazwischen einen Quetschspalt definieren, wobei mindestens die genannte erste Walze eine Mehrzahl von Umfangsrillen und Stegen zwischen den genannten Rillen aufweist;
Durchführen der genannten dünnen Blätter durch den genannten Quetschspalt in Gegenüberstellungsbeziehung, wobei das genannte erste dünne Blatt der genannnten ersten Walze gegenüberliegt;
querlaufendes Übereinstimmen der Falten des genannten gefältelten dünnen Blattes mit den genannten Rillen der genannten gerillten Walze;
Zusammenpressen der genannten Walzen, sodaß die genannten dünnen Blätter einander in einer den genannten Stegen entsprechenden Position berühren, um die genannten dünnen Blätter an den genannten Kontaktpositionen miteinander zu verbinden.

2. Ein Verfahren nach Anspruch 1, bei welchem das zweite dünne Blatt ungefältelt ist und bei welchem die zweite Walze keine Umfangsrillen und -stege aufweist;
wobei das Verfahren folgende Schritte umfaßt:
Durchführen der genannten dünnen Blätter durch den genannten Quetschspalt in Gegenüberstellungsbeziehung, wobei das genannte erste dünne Blatt der genannten ersten Walze gegenüberliegt und das genannte zweite dünne Blatt der genannten zweiten Walze gegenüberliegt.

3. Ein Verfahren nach Anspruch 1, wobei jedes dünne Blatt Falten aufweist und die genannten Falten der genannten dünnen Blätter im wesentlichen zueinander parallel und nach außen gewandt sind, bei welchem jede genannte Walze eine Mehrzahl von Umfangsrillen und Stegen zwischen den genannten Rillen aufweist, wobei mindestens ein Steg der genannten ersten Walze querlaufend übereingestimmt ist mit mindestens einem Steg der genannten zweiten Walze, wobei das Verfahren folgende Schritte umfaßt:
Durchführen der genannten dünnen Blätter durch den genannten Quetschspalt in Gegenüberstellungsbeziehung, wobei die genannten Falten gegenüberliegend angeordnet sind und die genannten Falten des genannten ersten dünnen Blattes den genannten Rillen der genannten ersten Walze gegenüberliegen und die genannten Falten des genannten zweiten dünnen Blattes den genannten Rillen der genannten zweiten Walze gegenüberliegen; und
querlaufendes Übereinstimmen der Falten des genannten ersten dünnen Blattes mit den genannten Rillen der genannten ersten Walze und der Falten des genannten zweiten dünnen Blattes mit den genannten Rillen der genannten zweiten Walze.

4. Das Verfahren nach Anspruch 1, 2 oder 3, bei welchem mindestens eines der genannten dünnen Blätter durch den genannten Quetschspalt bei einer Geschwindigkeit von etwa 120 Meter pro Minute bis etwa 310 Meter pro Minute durchgeführt wird.

5. Das Verfahren nach Anspruch 4, bei welchem die genannten dünnen Blätter durch den genannten Quetschspalt bei einer Geschwindigkeitsdifferenz von mindestens etwa 2 % der Geschwindigkeit des dünnen Blattes, welches die geringere Geschwindigkeit aufweist, durchgehen.

6. Das Verfahren nach Anspruch 1, 2 oder 3, bei welchem die genannten Walzen (22) und (24) zusammengepreßt werden, sodaß ein Druck von etwa 42,200,000 Kilogramm pro Quadratmeter bis etwa 56,200,000 Kilogramm pro Quadratmeter an mindestens einer Walze erhalten wird.

7. Das Verfahren nach Anspruch 1,2 oder 3, welches weiters den Schritt des Erhitzens von mindestens einer der genannten Walzen auf eine Temperatur, welche ausreichend ist, um das Verbinden der genannten dünnen Blätter zu bewirken, umfaßt.

8. Das Verfahren nach Anspruch 1, 2 oder 3, bei welchem jede der genannten gerillten Walzen eine Ganghöhe aufweist, welche aus der Gruppe bestehend aus einheitlicher Beabstandung und nicht-einheitlicher Beabstandung ausgewählt ist.

9. Das Verfahren nach Anspruch 1, 2 oder 3, welches weiters den Schritt des Dazwischenlegens eines dritten dünnen Blattes zwischen das genannte erste dünne Blatt und das genannte zweite dünne Blatt umfaßt, sodaß ein Laminat mit drei dünnen Blättern, welche in Gegenüberstellungsbeziehung verbunden werden, gebildet wird.

10. Das Verfahren nach Anspruch 1,2 oder 3, welches weiters den Schritt des Streckens von mindestens einem der genannten dünnen Blätter umfaßt.

## Revendications

1. Un procédé pour relier deux couches face à face, au moins l'une des couches ayant des plis, chaque couche étant constituée d'un matériau tissé ou non tissé souple, d'un matériau en film ou d'un matériau en feuille de papier, ledit procédé comprenant les étapes suivantes :
on fournit une première couche et une seconde couche , au moins ladite première couche ayant des plis ;
on fournit un premier rouleau et un second rouleau ayant des axes mutuellement parallèles et délimitant un pincement entre eux, au moins ledit premier rouleau ayant une pluralité de rainures circonférentielles et de surfaces de réception entre lesdites rainures;
on fait passer lesdites couches disposées face à face à travers ledit pincement, grâce à quoi ladite première couche est tournée vers ledit premier rouleau;
on fait coïncider transversalement les plis de ladite couche plissée avec lesdites rainures dudit rouleau rainuré;
on applique par pression lesdits rouleaux l'un contre l'autre de telle sorte que lesdites couches viennent en contact l'une avec l'autre en un endroit correspondant auxdites surfaces de réception pour relier ensemble lesdites couches dans lesdits endroits de contact.

2. Procédé selon la revendication 1, dans lequel la seconde couche n'est pas plissée et dans lequel le second rouleau ne comporte ni rainure circonférentielle ni surface de réception ;
Le procédé comprenant les étapes suivantes :
on fait passer lesdites couches à travers ledit pincement, face à face, grâce à quoi ladite première couche est tournée vers ledit premier rouleau et ladite seconde couche est tournée vers ledit second rouleau.

3. Procédé selon la revendication 1, chaque couche comportant des plis, lesdits plis desdites couches étant sensiblement mutuellement parallèles et tournés vers l'extérieur, dans lequel chacun desdits rouleaux comporte une pluralité de rainures circonférentielles et de surfaces de réception intercalées entre lesdites rainures, au moins l'une desdites surfaces de réception dudit premier rouleau coïncidant transversalement avec au moins l'une desdites surfaces de réception dudit second rouleau, le procédé comprenant les étapes suivantes :
on fait passer lesdites couches face à face à travers ledit pincement, grâce à quoi lesdits plis sont disposés en étant opposés et lesdits plis de ladite première couche sont tournés vers lesdites rainures dudit premier rouleau et lesdits plis de ladite seconde couche sont tournés vers lesdites rainures dudit second rouleau; et
on fait coïncider transversalement les plis de ladite première couche avec lesdites rainures dudit premier rouleau et les plis de ladite seconde couche avec lesdites rainures dudit second rouleau.

4. Procédé selon les revendications 1, 2 ou 3 dans lequel, au moins l'une desdites couches passe à travers ledit pincement à une vitesse d'environ 120 mètres par minute à environ 310 mètres par minute.

5. Procédé selon la revendication 4, dans lequel lesdites couches passent à travers ledit pincement à une vitesse différentielle d'au moins 2 % environ par rapport à la vitesse de la couche ayant la vitesse la plus basse.

6. Procédé selon les revendications 1, 2 ou 3 dans lequel, lesdits rouleaux 22 et 24 sont appliqués l'un contre l'autre par pression de telle sorte qu'une pression d'environ 42.200.000 kilogrammes par mètre carré à environ 56.200.000 kilogrammes par mètre carré est appliquée sur au moins l'un des rouleaux.

7. Procédé selon les revendications 1, 2 ou 3 comportant, en outre, l'étape consistant à chauffer au moins l'un desdits rouleaux à une température suffisante pour réaliser la liaison desdites couches.

8. Procédé selon les revendications 1, 2 ou 3, dans lequel chacun desdits rouleaux rainurés à un pas choisi à partir du type comportant un espacement uniforme et un espacement non uniforme.

9. Procédé selon les revendications 1, 2, ou 3, comportant, en outre, l'étape consistant à interposer une troisième couche entre ladite première couche et ladite seconde couche, de manière à former un stratifié à trois couches réunies face à face.

10. Procédé selon les revendications 1, 2 ou 3, comportant, en outre, l'étape consistant à étirer au moins l'une desdites couches.
